(19) Europäisches Patentamt
European Patent Office
Office européen des brevets

(11) EP 2 263 646 A1

(12) EUROPÄISCHE PATENTANMELDUNG

(43) Veröffentlichungstag:
**22.12.2010 Patentblatt 2010/51**

(21) Anmeldenummer: **10177608.6**

(22) Anmeldetag: **16.06.2008**

(51) Int Cl.:
***A61K 8/73*** *(2006.01)* ***A61K 8/81*** *(2006.01)*
***A61Q 5/06*** *(2006.01)*

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **09.11.2007 DE 102007053955**

(62) Dokumentnummer(n) der früheren Anmeldung(en) nach Art. 76 EPÜ:
**08761062.2 / 2 205 207**

(71) Anmelder: **Henkel AG & Co. KGaA**
**40589 Düsseldorf (DE)**

(72) Erfinder:
- **Knappe, Thorsten**
  **22869, Schenefeld (DE)**
- **Scheffler, Rene**
  **25479, Ellerau (DE)**

Bemerkungen:
Diese Anmeldung ist am 20-09-2010 als Teilanmeldung zu der unter INID-Code 62 erwähnten Anmeldung eingereicht worden.

# (54) Stylingmittel mit hohem Haltegrad bei Feuchtigkeit

(57) Mittel zur temporären Verformung keratinischer Fasern, die sich durch einen sehr hohen Haltegrad auszeichnen, ohne daß dabei auf Flexibilität und gute Feuchtebeständigkeit verzichtet werden müsste, enthalten in einem kosmetisch akzeptablen Träger
a) mindestens ein Copolymer A, gebildet aus
- mindestens einem Monomer A1 ausgewählt aus Acrylsäure und/oder Methacrylsäure, und
- mindestens einem Monomer A2 ausgewählt aus Acrylsäureestern und/oder Methacrylsäureestern

b) und mindestens ein von Copolymer A unterschiedliches kationisches Polymer B, ausgewählt aus Copolymeren von Vinylpyrrolidon mit Methacrylamidopropyltrimethylammoniumchlorid (MAPTAC).

EP 2 263 646 A1

**Beschreibung**

Die vorliegende Erfindung betrifft Mittel zur temporären Verformung keratinischer Fasern, enthaltend eine spezielle Kombination von Polymeren, die Verwendung dieser Mittel zur temporären Verformung keratinischer Fasern und Aerosolhaarschäume auf Basis dieser Mittel. Unter keratinischen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweis ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Stylingmittel zu entwickeln, die alle gewünschten Eigenschaften in Kombination aufweisen, bereitet nach wie vor Schwierigkeiten. Insbesondere gilt dies für Stylingmittel, die einen besonders starken Halt aufweisen sollen.

Aufgabe der vorliegenden Erfindung war es daher, ein Mittel zur temporären Verformung keratinischer Fasern zur Verfügung zu stellen, das sich durch einen sehr hohen Haltegrad auszeichnet ohne daß dabei auf Flexibilität und gute Feuchtebeständigkeit verzichtet werden müsste.

Es wurde nunmehr überraschenderweise gefunden, daß dies durch eine Kombination spezieller Polymere erreicht werden kann.

Ein erster Gegenstand der vorliegenden Erfindung ist daher ein kosmetisches Mittel, enthaltend in einem kosmetisch akzeptablen Träger

a) mindestens ein Copolymer A, gebildet aus

- mindestens einem Monomer A1 ausgewählt aus Acrylsäure und/oder Methacrylsäure, und
- mindestens einem Monomer A2 ausgewählt aus Acrylsäureestern und/oder Methacrylsäureestern

b) und mindestens ein von Copolymer A unterschiedliches kationisches Polymer B, ausgewählt aus Copolymeren von Vinylpyrrolidon mit Methacrylamidopropyltrimethylammoniumchlorid (MAPTAC).

Filmbildende und/oder festigende Copolymere A sind bekannt. Gleiches gilt für kationische Polymere B und ihre Verwendung als filmbildende und/oder festigende Polymere. Es hat sich nun überraschenderweise gezeigt, dass

eine entsprechende Kombination beider Polymertypen selbstverdickende Eigenschaften besitzt, wobei die ausgezeichneten filmbildenden und/oder festigenden Eigenschaften der einzelnen Polymere noch erhöht werden. Stylingmittel, enthaltend eine Kombination dieser Polymere, zeichnen sich durch eine synergistische Erhöhung des Haltegrads und gute Feuchtebeständigkeit des erzielten Halts aus, ohne dabei in ihrer Auswaschbarkeit beeinträchtigt zu werden.

Als ersten zwingenden Bestandteil enthalten die erfindungsgemäßen kosmetischen Mittel mindestens ein Copolymer A. Dieses Copolymer besitzt mindestens einen Monomerbaustein A1 ausgewählt aus Acrylsäure und/oder Methacrylsäure, und mindestens einen Monomerbaustein A2 ausgewählt aus Acrylsäureestern und/oder Methacrylsäureestern und kann darüber hinaus weitere Struktureinheiten aufweisen, die durch das Hinzufügen entsprechender Monomere bei der Polymerisation einpolymerisiert werden.

Besonders bevorzugt ist Monomer A1 ausgewählt aus Acrylsäure und/oder Methacrylsäure und Monomer A2 ausgewählt aus Acrylsäuremethylester, Methacrylsäuremethylester, Acrylsäureethylester, Methacrylsäureethylester, Acrylsäurepropylester, Methacrylsäurepropylester, Acrylsäureisopropylester, Methacrylsäureisopropylester, Acrylsäureoctyllester, Methacrylsäureoctylester, Acrylsäuredecylester, Methacrylsäureccdecylester, Acrylsäurelauyrlester, Methacrylsäurelaurylester, Acrylsäuremyristylester, Methacrylsäuremyristylester, Acrylsäurecetylester, Methacrylsäurecetylester, Acrylsäurestearylester, Methacrylsäurestearylester, Acrylsäureeicosylester und Methacrylsäureeicosylester, ganz besonders bevorzugt aus Acrylsäure, Methacrylsäure, Acrylsäuremethylester, Methacrylsäuremethylester, Acrylsäureethylester, Methacrylsäureethylester, Acrylsäurelaurylester, Methacrylsäurelaurylester, Acrylsäurestearylester und Methacrylsäurestearylester.

Besonders bevorzugte Copolymere A sind im Rahmen der vorliegenden Erfindung

- Copolymere von Acrylsäure mit Acrylsäuremethylester,
- Copolymere von Acrylsäure mit Methacrylsäuremethylester,
- Copolymere von Acrylsäure mit Acrylsäureethylester,
- Copolymere von Acrylsäure mit Methacrylsäureethylester,
- Copolymere von Acrylsäure mit Acrylsäurepropylester,
- Copolymere von Acrylsäure mit Methacrylsäurepropylester,
- Copolymere von Acrylsäure mit Acrylsäureisopropylester,
- Copolymere von Acrylsäure mit Methacrylsäureisopropylester,
- Copolymere von Acrylsäure mit Acrylsäureoctyllester,
- Copolymere von Acrylsäure mit Methacrylsäureoctylester,
- Copolymere von Acrylsäure mit Acrylsäuredecylester,
- Copolymere von Acrylsäure mit Methacrylsäureccdecylester,
- Copolymere von Acrylsäure mit Acrylsäurelauyrlester,
- Copolymere von Acrylsäure mit Methacrylsäurelaurylester,
- Copolymere von Acrylsäure mit Acrylsäuremyristylester,
- Copolymere von Acrylsäure mit Methacrylsäuremyristylester,
- Copolymere von Acrylsäure mit Acrylsäurecetylester,
- Copolymere von Acrylsäure mit Methacrylsäurecetylester,
- Copolymere von Acrylsäure mit Acrylsäurestearylester,
- Copolymere von Acrylsäure mit Methacrylsäurestearylester,
- Copolymere von Acrylsäure mit Acrylsäureeicosylester,
- Copolymere von Acrylsäure mit Methacrylsäureeicosylester
- Copolymere von Methacrylsäure mit Acrylsäuremethylester,
- Copolymere von Methacrylsäure mit Methacrylsäuremethylester,
- Copolymere von Methacrylsäure mit Acrylsäureethylester,
- Copolymere von Methacrylsäure mit Methacrylsäureethylester,
- Copolymere von Methacrylsäure mit Acrylsäurepropylester,
- Copolymere von Methacrylsäure mit Methacrylsäurepropylester,
- Copolymere von Methacrylsäure mit Acrylsäureisopropylester,
- Copolymere von Methacrylsäure mit Methacrylsäureisopropylester,
- Copolymere von Methacrylsäure mit Acrylsäureoctyllester,
- Copolymere von Methacrylsäure mit Methacrylsäureoctylester,
- Copolymere von Methacrylsäure mit Acrylsäuredecylester,
- Copolymere von Methacrylsäure mit Methacrylsäureccdecylester,
- Copolymere von Methacrylsäure mit Acrylsäurelauyrlester,
- Copolymere von Methacrylsäure mit Methacrylsäurelaurylester,
- Copolymere von Methacrylsäure mit Acrylsäuremyristylester,
- Copolymere von Methacrylsäure mit Methacrylsäuremyristylester,

- Copolymere von Methacrylsäure mit Acrylsäurecetylester,
- Copolymere von Methacrylsäure mit Methacrylsäurecetylester,
- Copolymere von Methacrylsäure mit Acrylsäurestearylester,
- Copolymere von Methacrylsäure mit Methacrylsäurestearylester,
- Copolymere von Methacrylsäure mit Acrylsäureeicosylester,
- Copolymere von Methacrylsäure mit Methacrylsäureeicosylester
- Copolymere von Acrylsäure und Methacrylsäure mit Acrylsäuremethylester,
- Copolymere von Acrylsäure und Methacrylsäure mit Methacrylsäuremethylester,
- Copolymere von Acrylsäure und Methacrylsäure mit Acrylsäureethylester,
- Copolymere von Acrylsäure und Methacrylsäure mit Methacrylsäureethylester,
- Copolymere von Acrylsäure und Methacrylsäure mit Acrylsäurepropylester,
- Copolymere von Acrylsäure und Methacrylsäure mit Methacrylsäurepropylester,
- Copolymere von Acrylsäure und Methacrylsäure mit Acrylsäureisopropylester,
- Copolymere von Acrylsäure und Methacrylsäure mit Methacrylsäureisopropylester,
- Copolymere von Acrylsäure und Methacrylsäure mit Acrylsäureoctyllester,
- Copolymere von Acrylsäure und Methacrylsäure mit Methacrylsäureoctylester,
- Copolymere von Acrylsäure und Methacrylsäure mit Acrylsäuredecylester,
- Copolymere von Acrylsäure und Methacrylsäure mit Methacrylsäureccdecylester,
- Copolymere von Acrylsäure und Methacrylsäure mit Acrylsäurelauyrlester,
- Copolymere von Acrylsäure und Methacrylsäure mit Methacrylsäurelaurylester,
- Copolymere von Acrylsäure und Methacrylsäure mit Acrylsäuremyristylester,
- Copolymere von Acrylsäure und Methacrylsäure mit Methacrylsäuremyristylester,
- Copolymere von Acrylsäure und Methacrylsäure mit Acrylsäurecetylester,
- Copolymere von Acrylsäure und Methacrylsäure mit Methacrylsäurecetylester,
- Copolymere von Acrylsäure und Methacrylsäure mit Acrylsäurestearylester,
- Copolymere von Acrylsäure und Methacrylsäure mit Methacrylsäurestearylester,
- Copolymere von Acrylsäure und Methacrylsäure mit Acrylsäureeicosylester und
- Copolymere von Acrylsäure und Methacrylsäure mit Methacrylsäureeicosylester.

  Besonders bevorzugte erfindungsgemäße Mittel sind dadurch gekennzeichnet, daß sie als Copolymer A ein Copolymer A1 aus Methacrylsäure und Ethylacrylat enthalten.

Vorzugsweise werden Copolymere A1 eingesetzt, die 10 bis 80 Mol-%, vorzugsweise 20 bis 70 Mol.-%, besonders bevorzugt 30 bis 60 Mol.-% und insbesondere 40 bis 50 Mol.-% Methacrylsäure und 20 bis 90 Mol.-%, vorzugsweise 30 bis 80 Mol.-%, besonders bevorzugt 40 bis 70 Mol.-% und insbesondere 50 bis 60 Mol.-% Ethylacrylat enthalten.

Noch weiter bevorzugt sind erfindungsgemäße Mittel, bei denen das Copolymer A1 eine Molmasse von 100 bis 800 kDa, vorzugsweise von 200 bis 700 kDa, weiter bevorzugt von 300 bis 600 kDa und insbesondere von 450 bis 550 kDa aufweist.

Besonders bevorzugt enthält das erfindungsgemäße Mittel das Copolymer, das von der Firma BASF AG unter der Bezeichnung Luviflex® Soft (INCI-Bezeichnung: Acrylates Copolymer) vertrieben wird.

Unabhängig davon, welche(s) Copolymer(e) A eingesetzt wird bzw. werden, sind erfindungsgemäße Mittel bevorzugt, bei denen die Gesamtmenge an Copolymeren A, bezogen auf das Gewicht des anwendungsbereiten Mittels, 0,05 bis 5 Gew.-%, vorzugsweise 0,1 bis 4 Gew.-% und insbesondere 0,25 bis 3 Gew.-%, beträgt.

Die erfindungsgemäßen Mittel enthalten mindestens ein weiteres Polymer B aus der Gruppe der kationischen Polymere, d.h. der Polymere, die mindestens eine Monomereinheit mit einer positiv geladenen Gruppe enthalten. Dieses kationische Polymer ist ausgewählt aus Copolymeren von Vinylpyrrolidon mit Methacrylamidopropyltrimethylammoniumchlorid (MAPTAC).

Diese lassen sich durch die allgemeine Formel

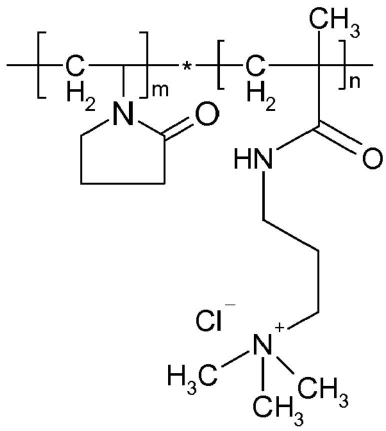

beschreiben, wobei die Indices m und n je nach Molmasse des Polymers variieren und nicht bedeuten sollen, daß es sich um Blockcopolymere handelt. Vielmehr können Struktureinheiten im Molekül statistisch verteilt vorliegen.

Besonders bevorzugte erfindungsgemäße Mittel sind **dadurch gekennzeichnet, daß** sie als kationisches Polymer B Copolymere von Methacrylamidopropyltrimethylammoniumchlorid (MAPTAC) mit Vinylpyrrolidon enthalten, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten. Besonders bevorzugte erfindungsgemäße Mittel sind weiter **dadurch gekennzeichnet, daß** die Copolymere b3 Molmassen von 10 bis 1000 kDa, vorzugsweise von 25 bis 900 kDa, weiter bevorzugt von 50 bis 800 kDa und insbesondere von 100 bis 750 kDa, aufweisen.

Vorzugsweise werden die Copolymere B innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) B enthalten.

Ein ganz besonders bevorzugtes Copolymer B wird nach der INCI-Nomenklatur als Polyquaternium-28 bezeichnet. Ein solches Polymer ist beispielsweise unter der Handelsbezeichnung Gafquat® HS-100 (ISP) erhältlich.

Hinsichtlich der Auswahl der Polymere A und B unterliegt die vorliegende Erfindung keinen Beschränkungen. Es können sowohl jeweils nur ein Polymer als auch jeweils mehrere Polymere aus den einzelnen beschriebenen Klassen eingesetzt werden. Besonders bevorzugte Mittel enthalten mindestens eine der unter den Nummern (1) bis (264) im Prioritätsdokument genannten Polymerkombinationen. Unabhängig davon, welche der 264 bevorzugten Polymerkombinationen gewählt wird, sind erfindungsgemäße Mittel bevorzugt, bei denen das Gewichtsverhältnis von Polymer(en) A zu Polymer(en) B 10:1 bis 1:10, vorzugsweise 8:1 bis 1:8, weiter bevorzugt 5:1 bis 1:5 und insbesondere 4:1 bis 1:4 beträgt.

Unabhängig von Art und Gewichtsverhältnis der Polymeren zueinander sind darüber hinaus erfindungsgemäße Mittel bevorzugt, bei denen der Gesamt-Polymergehalt der Mittel 1 bis 15 Gew.-%, vorzugsweise 2,5 bis 12,5 Gew.-%, weiter bevorzugt 4 bis 10 Gew.-% und insbesondere 5 bis 8 Gew.-% beträgt.

Die erfindungsgemäßen Mittel enthalten die Polymere in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol , Butylenglycol, Sorbitol und Propylenglykol in einer Menge bis 30 Gew.-% bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise den jeweiligen kosmetischen Mitteln zugesetzt werden.

Als geeignete Hilfs- und Zusatzstoffe sind insbesondere Pflegestoffe zu nennen. Diese finden sowohl bei Haut- als auch Haarbehandlungsmitteln Anwendung und können bei geeigneter Wahl des Pflegestoffs beispielsweise in Cremes, Shampoos, Haarspülungen, Haarkuren, Gele, Pump-und Aerosolsprays und Schaumprodukte eingearbeitet

werden.

Als Pflegestoff kann beispielsweise ein Silikonöl und/oder ein Silikongum eingesetzt werden. In einer besonderen Ausführungsform der Erfindung enthalten die Mittel mindestens ein Silikonöl und/oder ein Silikongum.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Silikonöle bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikonöle versteht der Fachmann mehrere Strukturen Siliciumorganischer Verbindungen. Zunächst werden hierunter die Dimethiconole (S1) verstanden. Diese können sowohl linear als auch verzweigt als auch cyclisch oder cyclisch und verzweigt sein. Lineare Dimethiconole können durch die folgende Strukturformel (S1 - I) dargestellt werden:

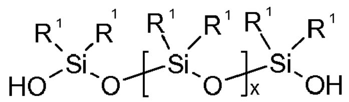

(S1 – I)

Verzweigte Dimethiconole können durch die Strukturformel (S1 - 11) dargestellt werden:

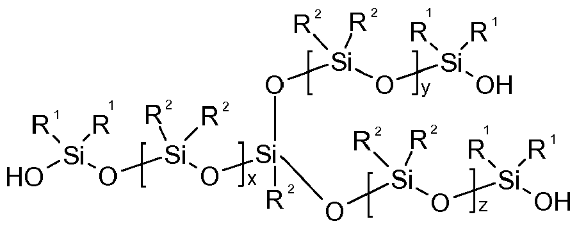

(S1 – II)

Die Reste $R^1$ und $R^2$ stehen unabhängig voneinander jeweils für Wasserstoff, einen Methylrest, einen $C_2$ bis $C_{30}$ linearen, gesättigten oder ungesättigten Kohlenwasserstoffrest, einen Phenylrest und/oder einen Arylrest. Nicht einschränkende Beispiele der durch $R^1$ und $R^2$ repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist $R^1$ und $R^2$ ein Alkylrest, der 1 bis etwa 6 Kohlenstoffatomen enthält, insbesondere bevorzugt ist $R^1$ und $R^2$ Methyl. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle

zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Dimethicone (S2) bilden die zweite Gruppe der Silikone, welche erfindungsgemäß enthalten sein können. Diese können sowohl linear als auch verzweigt als auch cyclisch oder cyclisch und verzweigt sein. Lineare Dimethicone können durch die folgende Strukturformel (S2 - I) dargestellt werden:

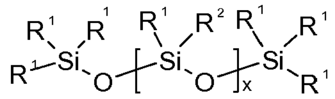

(S2 – I)

Verzweigte Dimethicone können durch die Strukturformel (S2 - 11) dargestellt werden:

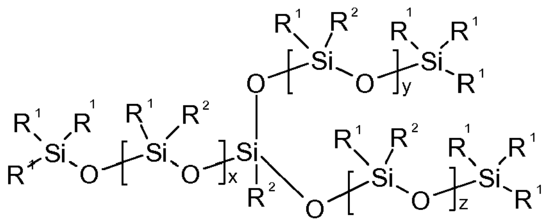

(S2 – II)

Die Reste $R^1$ und $R^2$ stehen unabhängig voneinander jeweils für Wasserstoff, einen Methylrest, einen $C_2$ bis $C_{30}$ linearen, gesättigten oder ungesättigten Kohlenwasserstoffrest, einen Phenylrest und/oder einen Arylrest. Nicht einschränkende Beispiele der durch $R^1$ und $R^2$ repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist $R^1$ und $R^2$ ein Alkylrest, der 1 bis etwa 6 Kohlenstoffatomen enthält, und besonders bevorzugt ist $R^1$ und $R^2$ Methyl. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, besonders bevorzugte Viskositäten liegen zwischen 10000 und 3000000 cPs. Ganz besonders bevorzugt liegt die Viskosität im Bereich zwischen 50000 und 2000000 cPs. Dimethiconcopolyole (S3) bilden eine weitere Gruppe von Silikonen, die geeignet sind. Dimethiconcopolyole können durch die folgenden Strukturformeln dargestellt werden:

$$(SiR^1_3) - O - (SiR^2_2 - O - )_x - (SiR^2PE - O - )_y - (SiR^1_3) \qquad (S3 - I),$$

$$PE - (SiR^1_2) - O - (SiR^2_2 - O - )_x -(SiR^1_2) - PE \qquad (S3 - II)$$

Verzweigte Dimethiconcopolyole können durch die Strukturformel (S3 - III) dargestellt werden:

$$\begin{array}{c} R^2 \\ | \\ PE - (SiR^1_2) - O - (SiR^2_2 - O - )_x - Si - O - (SiR^2_2 - O - )_y - (SiR^1_2) - PE \qquad (S3 - III) \\ | \\ O - (SiR^2_2 - O - )_z - (SiR^1_2) - PE \end{array}$$

oder durch die Strukturformel (S3 - IV):

$$\begin{array}{l} \qquad\qquad\qquad\qquad\qquad\qquad\qquad R^2 \\ \qquad\qquad\qquad\qquad\qquad\qquad\qquad | \\ (SiR^1_3) - O - (SiR^2_2 - O -)_x - Si - O - (SiR^2\ PE - O -)_y - (SiR^1_3) \\ \qquad\qquad\qquad\qquad\qquad\qquad\qquad | \\ \qquad\qquad\qquad\qquad\qquad\qquad\qquad O - (SiR^2_2 - O -)_z - (SiR^1_3) \end{array} \qquad (S3–IV)$$

Die Reste $R^1$ und $R^2$ stehen unabhängig voneinander jeweils für Wasserstoff, einen Methylrest, einen $C_2$ bis $C_{30}$ linearen, gesättigten oder ungesättigten Kohlenwasserstoffrest, einen Phenylrest und/oder einen Arylrest. Nicht einschränkende Beispiele der durch $R^1$ und $R^2$ repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist $R^1$ und $R^2$ ein Alkylrest, der 1 bis etwa 6 Kohlenstoffatomen enthält, insbesondere bevorzugt ist $R^1$ und $R^2$ Methyl. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Entsprechende Dimethiconcopolyole sind kommerziell erhältlich und werden beispielsweise von der Firma Dow Corning unter der Bezeichnung Dow Corning ® 5330 Fluid vertrieben. Selbstverständlich umfasst die erfindungsgemäße Lehre auch, dass die Dimethiconole, Dimethicone und/oder Dimethiconcopolymere bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt.

Wenn die Dimethiconole, Dimethicone und/oder Dimethiconcopolyole als Emulsion verwendet werden, dann beträgt die Tröpfchengröße der emulgierten Teilchen erfindungsgemäß 0,01 bis 10000 $\mu$m, bevorzugt 0,01 bis 100 $\mu$m, besonders bevorzugt 0,01 bis 20 $\mu$m und ganz besonders bevorzugt 0,01 bis 10 $\mu$m. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein. Geeignete Silikone sind weiterhin aminofunktionelle Silikone (S4), insbesondere die Silikone, die unter der INCI-Bezeichnung Amodimethicone zusammengefasst sind. Darunter sind Silikone zu verstehen, welche mindestens eine, gegebenenfalls substituierte, Aminogruppe aufweisen.

Solche Silikone können z.B. durch die Formel (S4 - I)

$$M(R_aQ_bSiO_{(4-a-b)/2)})_x(R_cSiO_{(4-c)/2)y})M \qquad (S4\ \text{-I})$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel - $R^1Z$ ist, worin $R^1$ eine zweiwertige, verbindende Gruppe ist, die an Wasserstoff und den Rest Z gebunden ist, zusammengesetzt aus Kohlenstoff- und Wasserstoffatomen,

Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silikon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2CH_2CH_2-$, $-CH_2CH(CH_3)C(O)OCH_2-$, $-(CH_2)_3C(O)OCH_2CH_2-$, $-C_6H_4C_6H_4-$, $-C_6H_4CH_2C_6H_4-$; und $-(CH_2)_3C(O)SCH_2CH_2-$ ein.

Z ist ein organischer, aminofunktioneller Rest, enthaltend mindestens eine funktionelle Aminogruppe. Eine mögliche Formel für Z ist $NH(CH_2)_zNH_2$, worin z für eine ganze Zahl von 1 bis 50 steht. Eine andere mögliche Formel für Z ist $-NH(CH_2)_zNH(CH_2)_{zz}$, worin sowohl z als auch zz unabhängig voneinander für eine ganze Zahl von 1 bis 50 stehen, wobei diese Struktur Diamino-Ringstrukturen umfasst, wie Piperazinyl. Z ist insbesondere bevorzugt ein $-NHCH_2CH_2NH_2$-Rest. Eine andere mögliche Formel für Z ist $-N(CH_2)_zNX^1X^2$ oder $-NX^1X^2$, worin $X^1$ und $X^2$ jeweils unabhängig voneinander ausgewählt ist aus Wasserstoff und einem Kohlenwasserstoffrest mit 1 bis etwa 6 Kohlenstoffatomen.

Ganz besonders bevorzugt steht Q für einen polaren, aminfunktionellen Rest der Formel $-CH_2CH_2CH_2NHCH_2CH_2NH_2$.

Das molare Verhältnis der $R_aQ_b\,SiO_{(4-a-b)/2}$-Einheiten zu den $R_cSiO_{(4-c)/2}$-Einheiten liegt im Bereich von etwa 1 : 2 bis 1 : 65, vorzugsweise von etwa 1 : 5 bis etwa 1 : 65 und besonders bevorzugt von etwa 1 : 15 bis etwa 1 : 20. Werden ein oder mehrere Silikone der obigen Formel eingesetzt, dann können die verschiedenen variablen Substituenten in der obigen Formel bei den verschiedenen Silikonkomponenten, die in der Silikonmischung vorhanden sind, verschieden sein.

Bevorzugte aminofunktionelle Silikone entsprechen der Formel (S4 - II)

$$R'_aG_{3-a}\text{-}Si(OSiG_2)_n\text{-}(OSiG_bR'_{2-b})_m\text{-}O\text{-}SiG_{3-a}\text{-}R'_a \qquad \text{(S4 - II)},$$

worin bedeutet:

- G ist -H, eine Phenylgruppe, -OH, $-O-CH_3$, $-CH_3$, $-CH_2CH_3$, $-CH_2CH_2CH_3$, $-CH(CH_3)_2$, $-CH_{2C}H_{2C}H_{2C}H_3$, $-CH_2CH(CH_3)_2$, $-CH(CH_3)CH_2CH_3$, $-C(CH_3)_3$ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
  o $-N(R'')-CH_2-CH_2-N(R'')_2$
  o $-N(R'')_2$
  o $-N'(R'')_3A^-$
  o $-N^+H(R'')_2\,A^-$
  o $-N^+H_2(R'')A^-$
  o $-N(R'')-CH_2-CH_2-N^+R''H_2A^-$,
  wobei jedes R" für gleiche oder verschiedene Reste aus der Gruppe -H, - Phenyl, -Benzyl, der $C_{1-20}$-Alkylreste, vorzugsweise $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$, steht und $A^-$ ein Anion repräsentiert, welches vorzugsweise
  ausgewählt ist aus Chlorid, Bromid, lodid oder Methosulfat.

Besonders bevorzugte aminofunktionelle Silikone entsprechen der Formel (S4 - III)

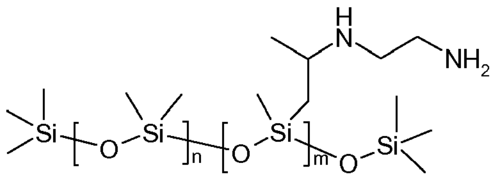

(S4 - III),

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet. Besonders bevorzugt sind weiterhin aminofunktionelle Silikone der Formel (S4 - IV)

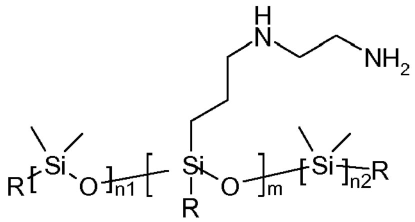

(S4 - IV),

worin R für -OH, $-O-CH_3$ oder eine $-CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone bezeichnet und sind beispielsweise in Form einer Emulsion als Handelsprodukt Dow Corning® 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Weitere geeignete Silikone sind beispielsweise

- oligomere Polydimethylcyclosiloxane (INCI-Bezeichnung: Cyclomethicone), insbesondere die tetramere und die pentamere Verbindung, die als Handelsprodukte DC 245 Fluid, DC 344 bzw. DC 345 von Dow Corning vertrieben werden,
- Hexamethyl-Disiloxan (INCI-Bezeichnung: Hexamethyldisiloxane), z. B. das unter der Bezeichnung Abil® K 520 vertriebenen Produkt,
- Polyphenylmethylsiloxane (INCI-Bezeichnung: Phenyl Trimethicone), z. B. das Handelsprodukt DC 556 Cosmetic Grade Fluid von Dow Corning,
- Ester sowie Partialester der Silikon-Glykol-Copolymere, wie sie beispielsweise von der Firma Fanning unter der Handelsbezeichnung Fancorsil® LIM (INCI-Bezeichnung: Dimethicone Copolyol Meadowfoamate) vertrieben werden,
- anionische Silikonöle, wie beispielsweise das Produkt Dow Corning®1784.

Gemäß einer bevorzugten Ausführungsform enthält das erfindungsgemäße Mittel mindestens zwei unterschiedliche Silikonderivate, insbesondere bevorzugt eine Kombination aus einem flüchtigen und einem nichtflüchtigen Silikon. Flüchtig im Sinne der Erfindung sind solche Silikone, die eine Flüchtigkeit aufweisen, die gleich oder größer als die Flüchtigkeit des cyclischen, pentameren Dimethylsiloxans ist. Solche Kombinationen sind auch als Handelsprodukte (z. B. Dow Corning®1401, Dow Corning®1403 und Dow Corning®1501, jeweils Mischungen aus einem Cyclomethicone

und einem Dimethiconol) erhältlich.

Bevorzugte Mischungen verschiedener Silikone sind beispielsweise Dimethicone und Dimethiconole, lineare Dimethicone und cylische Dimethiconole. Eine ganz besonders bevorzugte Mischung von Silikonen besteht aus mindestens einem cyclischen Dimethiconol und/oder Dimethicon, mindestens einem weiteren nicht cylischen Dimethicon und/oder Dimethiconol sowie mindestens einem aminofunktionellen Silikon.

Werden unterschiedliche Silikone als Mischung verwendet, so ist das Mischungsverhältnis weitgehend variabel. Bevorzugt werden jedoch alle zur Mischung verwendeten Silikone in einem Verhältnis von 5 : 1 bis 1 : 5 im Falle einer binären Mischung eingesetzt. Ein Verhältnis von 3 : 1 bis 1 : 3 ist besonders bevorzugt. Ganz besonders bevorzugte Mischungen enthalten alle in der Mischung enthaltenen Silikone weitestgehend in einem Verhältnis von etwa 1 : 1, jeweils bezogen auf die eingesetzten Mengen in Gew.-%.

Die Mittel enthalten die Silikone bevorzugt in Mengen von 1 - 25 Gew.-%, besonders bevorzugt von 5 - 20 Gew.-% und insbesondere bevorzugt von 7 - 15 Gew.-%, bezogen auf das gesamte Mittel.

Obwohl das erfindungsgemäße Mittel als Pflegestoff vorzugsweise ein Silikonderivat enthält, ist es auch möglich, dass das Mittel statt oder neben einer Silikonkomponente mindestens einen Pflegestoff einer anderen Verbindungsklasse enthält.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Erfindungsgemäß können Proteinhydrolysate sowohl pflanzlichen als auch tierischen oder marinen oder synthetischen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex), Sericin (Pentapharm) und Kerasol® (Croda) vertrieben. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.

Als Pflegestoff einer anderen Verbindungsklasse sind weiterhin kationische Tenside geeignet.

Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis (2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Als Pflegestoff eignen sich ebenfalls pflegende Polymere. Es sei an dieser Stelle darauf hingewiesen, dass

einige pflegende Polymere auch filmbildende und/oder festigende Eigenschaften aufweisen, und daher auch bei der Aufzählung geeigneter filmbildender und/oder festigender Polymere genannt sein können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I),

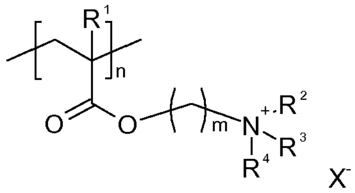

(G1-I)

in der $R^1$= -H oder -$CH_3$ ist, $R^2$, $R^3$ und $R^4$ unabhängig voneinander ausgewählt sind aus $C_{1-4}$-Alkyl-, -Alkenyl- oder -Hydroxyalkylgruppen, m = 1, 2, 3 oder 4, n eine natürliche Zahl und $X^-$ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

$R^1$ steht für eine Methylgruppe
$R^2$, $R^3$ und $R^4$ stehen für Methylgruppen
m hat den Wert 2.

Als physiologisch verträgliche Gegenionen $X^-$ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich. Copolymere mit Monomereinheiten gemäß Formel (G1-I) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,

- kationische Alkylpolyglycoside gemäß der DE-PS 44 13 686,
- kationisierter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylamino-modifiziertes Silikon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Luviquat®8155 und Luviquat® MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind.

Erfindungsgemäß bevorzugt eingesetzte kationische Polymere sind kationische Cellulose-Derivate und Chitosan und dessen Derivate, insbesondere die Handelsprodukte Polymer®JR 400, Hydagen® HCMF und Kytamer® PC, kationische Guar-Derivate, kationische Honig-Derivate, insbesondere das Handelsprodukt Honeyquat® 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17$^{th}$ Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus

(a) Monomeren mit quartären Ammoniumgruppen der allgemeinen Formel (II),

$$R^1\text{-CH=CR}^2\text{-CO-Z-}(C_nH_{2n})\text{-N(+)}R^3R^4R^5\ A^{(-)} \qquad \text{(II)}$$

in der $R^1$ und $R^2$ unabhängig voneinander stehen für Wasserstoff oder eine Methylgruppe und $R^3$, $R^4$ und $R^5$ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und $A^{(-)}$ das Anion einer organischen oder anorganischen Säure ist, und

(b) monomeren Carbonsäuren der allgemeinen Formel (III),

$$R^6\text{-CH=CR}^7\text{-COOH} \qquad \text{(III)}$$

in denen $R^6$ und $R^7$ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyltrimethylammoniumchlorid ist ein besonders bevorzugtes Monomeres (a). Als Monomeres (b) für die genannten Polymerisate wird bevorzugt Acrylsäure verwendet.

Die erfindungsgemäßen Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden. Panthenol, Pantolacton, Pyridoxin und seine Derivate sowie Nicotinsäureamid und Biotin sind besonders bevorzugt. Ganz besonders bevorzugt wird als Pflegestoff D-Panthenol, gegebenenfalls in Kombination mit mindestens einem der oben genannten Silikonderivate eingesetzt.

Als Pflegestoff können die erfindungsgemäßen Mittel weiterhin mindestens einen Pflanzenextrakt enthalten. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Grünem Tee, Mandel, Aloe Vera, Kokosnuss, Mango, Aprikose, Limone, Weizen, Kiwi und Melone.

Auch Mono- bzw. Oligosaccharide können als Pflegestoff in den erfindungsgemäßen Mitteln eingesetzt werden. Es können sowohl Monosaccharide als auch Oligosaccharide, wie beispielsweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten.

Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wird Glucose, die sowohl in der D-(+)- oder L-(-)- Konfiguration oder als Racemat geeignet ist. Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben.

Die erfindungsgemäßen Mittel enthalten die Lipide bevorzugt in Mengen von 0,01 - 10 Gew.-%, insbesondere 0,1 - 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,

- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I),

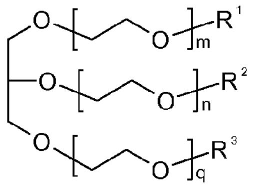

(D4-I)

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure
sowie deren technische Mischungen. Vorzugsweise werden Ölsäuremonoglyceride eingesetzt. Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Das Mittel kann überdies ein Enzym als Pflegestoff enthalten. Erfindungsgemäß besonders bevorzugte Enzyme sind ausgewählt aus einer Gruppe, die gebildet wird aus Proteasen, Lipasen, Transglutaminase, Oxidasen und Peroxidasen.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelte Haut oder die Haare vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In Abhängigkeit von der Art des erfindungsgemäßen Mittels kann es erforderlich sein, dass diese weiterhin mindestens ein Tensid enthalten. Dies gilt insbesondere für Hautreinigungsmittel und Shampoos. Aber auch andere Mittel, wie beispielsweise Haarspülungen, Haarkuren und bestimmte Stylingmittel, insbesondere Stylingschäume, können Tenside enthalten.

Beispielsweise können kationische Tenside eingesetzt werden, wie sie bereits oben als geeignete Pflegestoffe beschrieben sind. Bezüglich der bevorzugten kationischen Tenside und der eingesetzten Mengen gelten obige Ausführungen entsprechend.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische

Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel $R-O-(CH_2-CH_2O)_x-CH_2-COOH$, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel $R-O(CH_2-CH_2O)_x-OSO_3H$, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I),

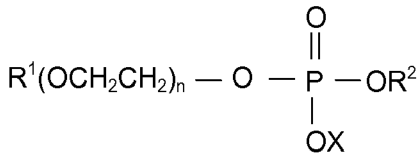

(E1-I)

in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^1$ oder X, n für Zahlen von 1 bis 10 und X für Wasserstoff, ein Alkali- oder Erdalkalimetall oder $NR^3R^4R^5R^6$, mit $R^3$ bis $R^6$ unabhängig voneinander stehend für Wasserstoff oder einen C1 bis C4 - Kohlenwasserstoffrest, steht,

- sulfatierte Fettsäurealkylenglykolester der Formel (E1-II)
  $R^7CO(AlkO)_nSO_3M$ (E1-II)
  in der $R^7CO$- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906 beschrieben sind,
- Monoglyceridsulfate und Monoglyceridethersulfate der Formel (E1-III)

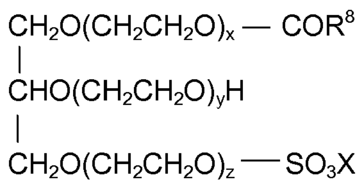

(E1-III)

in der $R^8CO$ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali-oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt,

in der $R^8C0$ für einen linearen Acylrest mit 8 bis 18 Kohlenstoffatomen steht,

- Amidethercarbonsäuren,
- Kondensationsprodukte aus $C_8$ - $C_{30}$ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer $C_8$ - $C_{24}$ - Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das $C_{12}$ - $C_{18}$ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

$$R^1CO-(OH_2CHR^2)_wOR^3 \qquad (E4-I)$$

in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,

- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$$R^4O-[G]_p \qquad (E4-II)$$

in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl-und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest $R^4$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkyloligoglucoside auf Basis technischer $C_{9/11}$-Oxoalkohole (DP = 1 bis 3). Der Alkyl- bzw. Alkenylrest $R^{15}$ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

- Zuckertenside vom Typ der Fettsäure-N-alkylpolyhydroxyalkylamide, ein nichtionisches Tensid der Formel (E4-III),

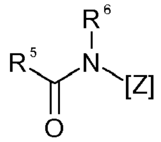

(E4-III)

in der $R^5CO$ für einen aliphatischen Acylrest mit 6 bis 22 Kohlenstoffatomen, $R^6$ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

$$R^7CO\text{-}NR^8\text{-}CH_2\text{-}(CHOH)_4CH_2OH \qquad \text{(E4-IV)}$$

Vorzugsweise werden als Fettsäure-N-alkylpolyhydroxyalkylamide Glucamide der Formel (E4-IV) eingesetzt, in der $R^8$ für Wasserstoff oder eine Alkylgruppe steht und $R^7CO$ für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.-% üblich.

- Die Mittel können weiterhin mindestens einen Emulgator enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeit der Emulsion.

Die Emulgatoren werden bevorzugt in Mengen von 0,1 bis 25 Gew.-%, insbesondere 0,1 bis 3 Gew.-%, bezogen auf die jeweilige gesamte Zusammensetzung, eingesetzt.

Bevorzugt sind nichtionogene Emulgatoren mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen. Nichtionogene Emulgatoren mit einem HLB-Wert von 10 bis 16 sind erfindungsgemäß besonders bevorzugt.

Die Mittel können neben den genannten Komponenten weiterhin alle für entsprechende kosmetische Mittel bekannten Wirk-, Zusatz- und Hilfsstoffe enthalten.

Weitere Wirk-, Hilfs- und Zusatzstoffe, sind beispielsweise

- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate, Silikate (z.B. Laponite® XLG), gelbildende Verdicker (z.B. Structure® 2001, Synthylen® 2000),
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und

Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, $N_2O$, Dimethylether, $CO_2$ und Luft,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für kosmetische Mittel üblichen Formen erfolgen, beispielsweise in Form von Lösungen, die als Gesichts- oder Haarwasser oder Pump- oder Aerosolspray auf die Haut oder das Haar aufgebracht werden können, in Form von Cremes, Emulsionen, Wachsen, Gelen oder auch tensidhaltigen schäumenden Lösungen oder anderen Zubereitungen, die für die Anwendung auf der Haut oder dem Haar geeignet sind. Vorzugsweise handelt es sich jedoch bei den erfindungsgemäßen Mitteln um Mittel zur temporären Verformung keratinischer Fasern, d.h. um Stylingmittel. Bevorzugte Stylingmittel sind Stylinggele, Pumphaarsprays, Aerosolhaarspray, Pumphaarschäume und Aerosolhaarschäume.

Die Formulierung der erfindungsgemäßen Mittel kann in allen für kosmetische Mittel üblichen Formen erfolgen, beispielsweise in Form von Lösungen, die als Gesichts- oder Haarwasser oder Pump- oder Aerosolspray auf die Haut oder das Haar aufgebracht werden können, in Form von Cremes, Emulsionen, Wachsen, Gelen oder auch tensidhaltigen schäumenden Lösungen oder anderen Zubereitungen, die für die Anwendung auf der Haut oder dem Haar geeignet sind. Vorzugsweise handelt es sich jedoch bei den erfindungsgemäßen Mitteln um Mittel zur temporären Verformung keratinischer Fasern, d.h. um Stylingmittel. Bevorzugte Stylingmittel sind Stylinggele, Pumphaarsprays, Aerosolhaarspray, Pumphaarschäume und Aerosolhaarschäume.

Stylinggele ist dabei im Rahmen der vorliegenden Anmeldung der Oberbegriff für klare oder trübe Produkte, Stylingwachse, Stylingcremes, Stylinglotionen, Styling-Jellys usw. Letztlich fallen unter diesen Begriff alle Mittel zum Frisieren von Haaren, die nicht Haarsprays oder Schäume sind. Unter Haarschäumen werden dabei Zusammensetzungen verstanden, die bei der Entnahme aus einem geeigneten Behälter einen Schaum ausbilden. Es kann notwendig sein, den Mitteln Inhaltsstoffe zuzusetzen, die die Schaumbildung fördern oder einmal gebildeten Schaum stabilisieren. Insbesondere eignen sich dafür Tenside und/oder Emulgatoren, wie sie bereits oben beschrieben wurden. Vorzugsweise werden Tenside aus der Gruppe der kationischen Tenside eingesetzt. Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt. Da die erfindungsgemäß eingesetzte Polymerkombination jedoch selbstverdickende Eigenschaften aufweist, ist die Zugabe weiterer Strukturanten und/oder verdickender Polymere nicht zwingend erforderlich. Vorzugsweise enthalten die erfindungsgemäßen Mittel keine weiteren Strukturanten und/oder verdickender Polymere.

Sofern es sich bei den erfindungsgemäßen Mitteln um ein Aerosolprodukt handelt, enthält dieses zwingend ein Treibmittel.

Erfindungsgemäß geeignete Treibmittel sind beispielsweise $N_2O$, Dimethylether, $CO_2$ Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen.

Bevorzugt werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen

von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Ein zweiter Gegenstand der Erfindung ist daher ein Verfahren, bei dem das erfindungsgemäße, kosmetisches Mittel auf das Haar als Pumphaarspray, Aerosolhaarspray, Pumphaarschaum, Aerosolhaarschaum oder Stylinggel aufgetragen wird und gegebenenfalls mit den Handflächen und/oder den Fingern in das Haar eingearbeitet wird.

Die gewünschte Verformung der Haare kann dabei mit den Finger oder Händen sowie mit geeigneten, herkömmlichen Hilfsmitteln wie beispielsweise Kamm oder Bürste erfolgen.

Ein dritter Gegenstand der Erfindung ist die Verwendung der erfindungsgemäßen Mittel zur temporären Verformung keratinischer Fasern.

Die erfindungsgemäßen Mittel und Produkte, die diese Mittel enthalten, zeichnen sich insbesondere dadurch aus, dass sie behandeltem Haar einen sehr starken und feuchtebeständigen Frisurenhalt verleihen.

Ein weiterer Gegenstand der vorliegenden Erfindung ist ein Verfahren, bei dem ein erfindungsgemäßes kosmetisches Mittel auf das Haar als Pumphaarspray, Aerosolhaarspray, Pumphaarschaum, Aerosolhaarschaum oder Stylinggel aufgetragen wird und gegebenenfalls mit den Handflächen und/oder den Fingern in das Haar eingearbeitet wird.

Für das erfindungsgemäße Verfahren gilt mutatis mutandis das zu den erfindungsgemäßen Mitteln Gesagte.

Ein weiterer Gegenstand der vorliegenden Erfindung ist die Verwendung von Polymermischungen, enthaltend

a) mindestens ein Copolymer A, gebildet aus

- mindestens einem Monomer A1 ausgewählt aus Acrylsäure und/oder Methacrylsäure, und
- mindestens einem Monomer A2 ausgewählt aus Acrylsäureestern und/oder Methacrylsäureestern

b) und mindestens ein von Copolymer A unterschiedliches kationisches Polymer B, ausgewählt aus Copolymeren von Vinylpyrrolidon mit Methacrylamidopropyltrimethylammoniumchlorid (MAPTAC).

zur Verbesserung des Frisurenhaltes, insbesondere bei erhöhter Luftfeuchtigkeit.

Auch für die erfindungsgemäße Verwendung gilt mutatis mutandis das zu den erfindungsgemäßen Mitteln Gesagte.

Der Halt der Verformung, auch als Frisurenhalt bezeichnet, sowie Flexibilität, Elastizität und Plastizität werden dabei im Sinne der vorliegenden Erfindung nach der Omega-Loop Methode bestimmt.

Dazu wird eine trockene Haarsträhne (Euro-Naturhaar der Firma Kerling, Klebetresse dicht, einseitig geklebt, Gesamtlänge 150 mm, freie Länge 130 mm, Breite 10 mm, Gewicht 0,9 ± 0,1 g) für 30 Sekunden bis zum unteren Rand der Abklebung in die zu untersuchende Polymerlösung getaucht. Anschließend wird die überschüssige Lösung zwischen Daumen und Zeigefinger abgestrichen, so dass 0,5 ± 0,02 g der Lösung auf dem Haar verbleiben. Die mit der zu untersuchenden Lösung gesättigte Haarsträhne wird um einen Teflon-Zylinder mit einem Durchmesser von 36 mm gewickelt und die überstehenden Enden werden mit einem Clip fixiert. Die präparierten Strähnen werden anschließend über Nacht bei 25°C und 50% relativer Luftfeuchte oder bei 25°C und 75% relativer Luftfeuchte im Klimaschrank getrocknet und konditioniert.

Die konditionierte Strähne wird vorsichtig von dem Teflon-Zylinder entfernt. Der entstandene Ω-Loop, eine ringförmige Struktur des in seiner Form durch den ausgebildeten Polymerfilm stabilisierten Haars, wird in den an der Messdose befestigten Greifer eingespannt und bis dicht über die Bodenplatte eines Universalprüfgeräts AMETEK LF Plus der Firma AMETEK Precision Instuments Europe GmbH, Produktgruppe Lloyd abgesenkt. Die gesamte Messung erfolgt im Klimaschrank unter konstanten klimatischen Bedingungen bei 25°C und 50% relativer Luftfeuchte oder bei 25°C und 75% relativer Luftfeuchte.

Um standardisierte Ausgangsbedingungen zu schaffen, startet die Messung mit dem Anfahren einer Vorlast von 0,07 N mit einer Geschwindigkeit von 30 mm $min^{-1}$. Anschließend wird der Ω-Loop mit einer Geschwindigkeit von 60 mm $min^{-1}$ um 8 mm gestaucht, wobei die dazu nötige Kraft gemessen wird. Nachdem die charakteristische Kraft $F_1$ bei der maximalen Deformation von 8 mm aufgezeichnet wurde, wird die Strähne mit 60 mm $min^{-1}$ soweit entlastet, dass sie 10 mm von der Bodenplatte abhebt. Von hier aus beginnt der nächste Zyklus, indem erneut die Vorlast von 0,07 N angefahren und die Strähne anschließend um 8 mm gestaucht wird, hierbei gelten die gleichen Geschwindigkeiten wie oben beschrieben. Die Messung eines Ω-Loops umfasst insgesamt 10 Zyklen.

Mit dieser Messmethode lassen sich vier charakteristische Parameter zur Beschreibung der mechanischen Eigenschaften von filmbildenden Polymeren bestimmen. Halt, Flexibilität, Plastizität und Elastizität lassen sich nach folgenden Formeln aus den gemessenen Kräften berechnen:

$$Halt = F_1 \, [N]$$

($F_1$ entspricht der Maximalkraft der Messung)

$$Flexibilität = \frac{F_{10}}{F_1}$$

(gibt das Verhältnis der Maximalkräfte des zehnten zum ersten Zyklus an)

$$Plastizität = \frac{2 \cdot H_1 - H_{10}}{H_1}$$

(mit $H_1$ = 9 mm und $H_{10}$ = 9 mm + dauerhafte plastische Verformung der Strähne)

$$Elastizität = \frac{\dfrac{F_{10}(2mm) - F_{10}(1{,}5mm)}{0{,}5}}{\dfrac{F_1(2mm) - F_1(1{,}5mm)}{0{,}5}} = \frac{E_{10}}{E_1}$$

(zur Berechnung der Elastizität werden aus dem ersten und zehnten Zyklus jeweils die Kräfte zur Verformung um 1,5 mm und 2 mm erfasst und miteinander ins Verhältnis gesetzt).

**Beispiele**

Die folgenden Mengenangaben verstehen sich - soweit nichts anderes vermerkt ist - in Gewichtsprozent.

1 Produkt

Es wurden die erfindungsgemäßen Stylingmittel A bis D gemäß folgender Tabelle hergestellt.

| Rohstoffe | B |
|---|---|
| 2-Amino-2-Methylpropanol, 95% | 1,11 |
| Luviflex Soft [1] | 8,15 |
| Gafquat HS-100 [3] | 12,5 |

(fortgesetzt)

| Rohstoffe | B |
|---|---|
| Wasser, vollentsalzt | ad 100 |

[1] Copolymer aus Ethylacrylat und Methacrylsäure, 30% Dispersion in Wasser (INCI-Bezeichnung: Acrylates Copolymer) (BASF)
[3] Copolymerisat aus Vinylpyrrolidon, Methacrylamidopropyltrimethylammoniumchlorid (MAPTAC) (ca. 20 Gew.-% Festkörper in Wasser; INCI-Bezeichnung: Polyquaternium-28) (ISP)

2 Wirknachweis

Mit der Omega-Loop Methode (50 % bzw. 75 % relative Luftfeuchtigkeit, 25°C) wurde der Halt, die Flexibilität, die Elastizität und die Plastizität bestimmt, die sich beim Aufbringen verschiedener Poymere auf menschliches Haar erzielen lassen.

Die erfindungsgemäßen Stylingmittel B enthalten die Polymere(bezogen auf reines Polymer) in Mengen von jeweils 5 Gew.-%.

Als Vergleichsformulierungen wurden zunächst zunächst die nicht-erfindungsgemäßen Stylingmittel b1 und b2 gemäß folgenden Tabelle hergestellt:

| Rohstoffe | b1 | b2 |
|---|---|---|
| 2-Amino-2-Methylpropanol, 95% | 2,21 | 0,01 |
| Luviflex Soft [1] | 16,30 | - |
| Gafquat HS-100 [3] | - | 25,00 |
| Wasser, vollentsalzt | ad 100 | ad 100 |

Diese enthielten jeweils nur ein Polymer, das in aktivsubstanzgleichen Mengen eingesetzt wurde (5 Gew.-%).

Die Vergleichsbeispiele b1 und b2 wurden mit der Omega-Loop Methode gemessen. Die erhaltenen Werte wurden als Grundlage für die Berechnung eines Erwartungswertes für Kombinationen von zwei Polymeren genutzt. Ergibt beispielsweise die Formulierung b1 einen Wert von 3 und die Formulierung b2 einen Wert von 1, so liegt der Erwartungswert für die entsprechende 1:1-Mischung (erfindungsgemäßes Gel A) bei (3 + 1)/2 = 2.

Danach wurden die Polymermischungen mit der Omega-Loop Methode bei 50 % rel. Luftfeuchtigkeit und bei 75 % rel. Luftfeuchtigkeit gemessen und die Abweichung vom Erwartungswert berechnet. Aus den Quotienten der Werte bei 75 % rel. Luftfeuchtigkeit zu den Werten bei 50 % rel. Luftfeuchtigkeit lassen sich die Feuchtestabilitäten ermitteln. Die erhaltenen Ergebnisse sind in folgender Tabelle wiedergegeben:

| | Halt [N] | Flexibilität | Elastizität | Plastizität |
|---|---|---|---|---|
| **B** | > 10 % besser | > 20 % besser | > 20 % besser | gleich |

Die Ergebnisse zeigen, daß die Polymerkombinationen im Vergleich zum Erwartungswert deutlich bessere Ergebnisse zeigen und dabei sowohl bei einer Versuchsdurchführung bei 50 % relativer Luftfeuchtigkeit, als auch bei einer Versuchsdurchführung bei 75 % relativer Luftfeuchtigkeit deutlich verbesserte Haltegrade aufweisen, wobei Flexibilität, Elastizität und Plastizität vergleichbar oder sogar besser sind. Die erfindungsgemäßen Polymerkombinationen zeigen damit unerwartete synergistische Effekte.

**Patentansprüche**

**1.** Kosmetisches Mittel, enthaltend in einem kosmetisch akzeptablen Träger

a) mindestens ein Copolymer A, gebildet aus

- mindestens einem Monomer A1 ausgewählt aus Acrylsäure und/oder Methacrylsäure, und
- mindestens einem Monomer A2 ausgewählt aus Acrylsäureestern und/oder Methacrylsäureestern

b) und mindestens ein von Copolymer A unterschiedliches kationisches Polymer B, ausgewählt aus Copolymeren von Vinylpyrrolidon mit Methacrylamidopropyltrimethylammoniumchlorid (MAPTAC).

**2.** Mittel nach Anspruch 1, **dadurch gekennzeichnet, daß** es als Copolymer A ein Copolymer A1 aus Methacrylsäure und Ethylacrylat enthält, das 10 bis 80 Mol-%, vorzugsweise 20 bis 70 Mol.-%, besonders bevorzugt 30 bis 60 Mol.-% und insbesondere 40 bis 50 Mol.-% Methacrylsäure und 20 bis 90 Mol.-%, vorzugsweise 30 bis 80 Mol.-%, besonders bevorzugt 40 bis 70 Mol.-% und insbesondere 50 bis 60 Mol.-% Ethylacrylat enthält.

**3.** Mittel nach einem der Ansprüche 1 oder 2, **dadurch gekennzeichnet, daß** die Gesamtmenge an Copolymeren A, bezogen auf das Gewicht des anwendungsbereiten Mittels, 0,05 bis 5 Gew.-%, vorzugsweise 0,1 bis 4 Gew.-% und insbesondere 0,25 bis 3 Gew.-%, beträgt.

**4.** Mittel nach einem der Ansprüche 1 bis 3, **dadurch gekennzeichnet, daß** es als kationisches Polymer B Copolymere von Methacrylamidopropyltrimethylammoniumchlorid (MAPTAC) mit Vinylpyrrolidon enthält, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten.

**5.** Mittel nach einem der Ansprüche 1 bis 4, **dadurch gekennzeichnet, daß** es, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) B enthält.

**6.** Mittel nach einem der Ansprüche 1 bis 5, **dadurch gekennzeichnet, daß** das Gewichtsverhältnis von Potymer(en) A zu Polymer(en) B 10:1 1 bis 1:10, vorzugsweise 8:1 bis 1:8, weiter bevorzugt 5:1 bis 1:5 und insbesondere 4:1 bis 1:4 beträgt.

**7.** Mittel nach einem der Ansprüche 1 bis 6, **dadurch gekennzeichnet, daß** der Gesamt-Polymergehalt der Mittel 1 bis 15 Gew.-%, vorzugsweise 2,5 bis 12,5 Gew.-%, weiter bevorzugt 4 bis 10 Gew.-% und insbesondere 5 bis 8 Gew.-% beträgt.

**8.** Verwendung von Polymermischungen, enthaltend

a) mindestens ein Copolymer A, gebildet aus

- mindestens einem Monomer A1 ausgewählt aus Acrylsäure und/oder Methacrylsäure, und
- mindestens einem Monomer A2 ausgewählt aus Acrylsäureestern und/oder Methacrylsäureestern

b) und mindestens ein von Copolymer A unterschiedliches kationisches Polymer B, ausgewählt aus Copolymeren von Vinylpyrrolidon mit Methacrylamidopropyltrimethylammoniumchlorid (MAPTAC).
zur Verbesserung des Frisurenhaltes, insbesondere bei erhöhter Luftfeuchtigkeit.

Europäisches Patentamt
European Patent Office
Office européen des brevets

# EUROPÄISCHER RECHERCHENBERICHT

Nummer der Anmeldung
EP 10 17 7608

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 102 47 694 A1 (BEIERSDORF AG [DE])<br>22. April 2004 (2004-04-22)<br>* Ansprüche 1,9,13,14 *<br>* Seite 2, Absatz 6 *<br>* Seite 2, Absatz 10 *<br>* Seite 3, Absatz 23-26 *<br>* Seite 4, Zeilen 6,7 *<br>* Seite 4, Absatz 28 *<br>* Seite 4, Absatz 34 *<br>* Seite 12; Beispiele 1-6 *<br>* Seiten 6-8, Absätze 50,51 *<br>----- | 1-9 | INV.<br>A61K8/73<br>A61K8/81<br>A61Q5/06 |
| Y | REINHARDT: "Hair fixation: A new high-performance polymer"<br>PARFUMS, COSMETIQUES, AROMES, SOCIETE D'EXPANSION TECHNIQUE ET ECONOMIQUE S.A. PARIS, FR,<br>Bd. 123, 1. Januar 1995 (1995-01-01), Seiten 56-61, XP009115376<br>ISSN: 0337-3029<br>* das ganze Dokument *<br>----- | 1-9 | |
| | | | RECHERCHIERTE SACHGEBIETE (IPC) |
| Y | DE 43 14 305 A1 (BASF AG [DE])<br>3. November 1994 (1994-11-03)<br>* das ganze Dokument *<br>----- | 1-9 | A61K<br>A61Q |
| Y | VLACHY ET AL: "Solubilization of methacrylic acid based polymers by surfactants in acidic solutions"<br>JOURNAL OF COLLOID AND INTERFACE SCIENCE, ACADEMIC PRESS, NEW YORK, NY, US,<br>Bd. 315, Nr. 2,<br>25. September 2007 (2007-09-25), Seiten 445-455, XP022267663<br>ISSN: 0021-9797<br>* Zusammenfassung *<br>-----<br>-/-- | 1-9 | |

Der vorliegende Recherchenbericht wurde für alle Patentansprüche erstellt

1

| Recherchenort | Abschlußdatum der Recherche | Prüfer |
|---|---|---|
| München | 5. November 2010 | Grillenberger, Sonja |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 10 17 7608

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | FR 2 709 954 A (OREAL [FR]) 24. März 1995 (1995-03-24) * Ansprüche 1,4,7,15,16,18 * * Seiten 3-4 * * Seite 9; Beispiel 2 * * Seite 10; Beispiel 5 * * Seite 12; Beispiel 7 * ----- | 1-9 | |
| A | EP 1 712 256 A (BEIERSDORF AG [DE]) 18. Oktober 2006 (2006-10-18) * Seite 3, Absatz 13 * * Seite 3, Absatz 22 * * Seite 4, Absatz 27 * * Seite 6; Beispiel 5 * ----- | 1-9 | |
| A | WO 98/19653 A (PROCTER & GAMBLE [US]; NAMBU TAKANORI [JP]) 14. Mai 1998 (1998-05-14) * Ansprüche 1-3 * * Seite 1, Zeilen 8,19-32 * * Seite 9, Zeile 31 - Seite 10, Zeile 2 * * Seite 10, Zeilen 2-12 * * Seite 15, Zeilen 22-25 * ----- | 1-9 | RECHERCHIERTE SACHGEBIETE (IPC) |
| A | DE 37 16 381 A1 (OREAL [FR]) 19. November 1987 (1987-11-19) * Ansprüche 1,3,7,10,15,16 * * Seite 3, Zeilen 39-42 * * Seite 4, Zeilen 37-41 * * Seite 4, Zeile 44 - Seite 5, Zeile 3 * * Seite 4, Zeile 61 * * Seiten 6-9; Beispiele 1,4,9,10,13 * * Seite 7, Zeilen 44-55 * ----- | 1-9 | |
| | -/-- | | |

Der vorliegende Recherchenbericht wurde für alle Patentansprüche erstellt

1

| Recherchenort | Abschlußdatum der Recherche | Prüfer |
|---|---|---|
| München | 5. November 2010 | Grillenberger, Sonja |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 10 17 7608

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2006/130373 A (PROCTER & GAMBLE [US]; WELLA AG [DE]; SCHMENGER JURGEN [DE]; KUJAWA JO) 7. Dezember 2006 (2006-12-07)<br>* Anspruch 1 *<br>* Seite 1, Zeilen 24-28 *<br>* Seiten 13-19; Beispiele 1-7 *<br>* Seiten 21-23; Beispiele 8,9 *<br>----- | 1-9 | |
| A | EP 1 591 103 A (OREAL [FR])<br>2. November 2005 (2005-11-02)<br>* Seite 8; Beispiel 1 *<br>----- | 1-9 | |
| A | FR 2 816 833 A (OREAL [FR])<br>24. Mai 2002 (2002-05-24)<br>* Seite 15; Beispiel Mo *<br>----- | 1-9 | |
| A | EP 1 348 420 A (OREAL [FR])<br>1. Oktober 2003 (2003-10-01)<br>* Ansprüche 1,3,6,14,16 *<br>* Seite 2, Absätze 2,5 *<br>* Seite 12, Zeile 55 *<br>* Seite 12; Beispiele 2,3 *<br>----- | 1-9 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| München | 5. November 2010 | Grillenberger, Sonja |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..........
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 10 17 7608

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-11-2010

| Im Recherchenbericht angeführtes Patentdokument | Datum der Veröffentlichung | Mitglied(er) der Patentfamilie | Datum der Veröffentlichung |
|---|---|---|---|
| DE 10247694 A1 | 22-04-2004 | KEINE | |
| DE 4314305 A1 | 03-11-1994 | AT 159422 T | 15-11-1997 |
| | | DK 696916 T3 | 15-12-1997 |
| | | WO 9424986 A1 | 10-11-1994 |
| | | EP 0696916 A1 | 21-02-1996 |
| | | ES 2109692 T3 | 16-01-1998 |
| | | GR 3025736 T3 | 31-03-1998 |
| | | JP 8509210 T | 01-10-1996 |
| | | JP 2007176947 A | 12-07-2007 |
| | | US 6482393 B1 | 19-11-2002 |
| FR 2709954 A | 24-03-1995 | KEINE | |
| EP 1712256 A | 18-10-2006 | DE 102005017463 A1 | 19-10-2006 |
| WO 9819653 A | 14-05-1998 | AT 224695 T | 15-10-2002 |
| | | DE 69624007 D1 | 31-10-2002 |
| | | DE 69624007 T2 | 05-06-2003 |
| | | EP 0938284 A1 | 01-09-1999 |
| | | JP 3045402 B2 | 29-05-2000 |
| | | JP 11500463 T | 12-01-1999 |
| DE 3716381 A1 | 19-11-1987 | BE 1000398 A3 | 22-11-1988 |
| | | CA 1299301 C | 21-04-1992 |
| | | CH 672793 A5 | 29-12-1989 |
| | | DK 244087 A | 17-11-1987 |
| | | ES 2005574 A6 | 16-03-1989 |
| | | FR 2598611 A1 | 20-11-1987 |
| | | GB 2193501 A | 10-02-1988 |
| | | IT 1211336 B | 18-10-1989 |
| | | JP 2097348 C | 02-10-1996 |
| | | JP 8009528 B | 31-01-1996 |
| | | JP 62277311 A | 02-12-1987 |
| | | LU 86429 A1 | 16-12-1987 |
| | | NL 8701174 A | 16-12-1987 |
| | | SE 465351 B | 02-09-1991 |
| | | SE 8702007 A | 17-11-1987 |
| | | US 4839166 A | 13-06-1989 |
| WO 2006130373 A | 07-12-2006 | DE 202005008307 U1 | 21-07-2005 |
| | | EP 1888023 A1 | 20-02-2008 |
| | | US 2007006397 A1 | 11-01-2007 |
| EP 1591103 A | 02-11-2005 | AT 469638 T | 15-06-2010 |
| | | ES 2345446 T3 | 23-09-2010 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 10 17 7608

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-11-2010

| Im Recherchenbericht angeführtes Patentdokument | Datum der Veröffentlichung | Mitglied(er) der Patentfamilie | Datum der Veröffentlichung |
|---|---|---|---|
| EP 1591103 A | | FR 2869534 A1 | 04-11-2005 |
| FR 2816833 A | 24-05-2002 | AU 1834502 A | 03-06-2002 |
| | | WO 0241856 A1 | 30-05-2002 |
| EP 1348420 A | 01-10-2003 | AT 400249 T | 15-07-2008 |
| | | BR 0300817 A | 14-09-2004 |
| | | CA 2423811 A1 | 28-09-2003 |
| | | CN 1448124 A | 15-10-2003 |
| | | ES 2307879 T3 | 01-12-2008 |
| | | FR 2837699 A1 | 03-10-2003 |
| | | JP 2003300820 A | 21-10-2003 |
| | | KR 20030078764 A | 08-10-2003 |
| | | MX PA03002634 A | 11-08-2004 |
| | | PL 359384 A1 | 06-10-2003 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE PS4413686 C
- DE OS19756454 A
- DE OS19738866 A


### In der Beschreibung aufgeführte Nicht-Patentliteratur


- Römpp-Lexikon Chemie. Georg Thieme Verlag Stuttgart, 1997, 1764